# EUROPEAN PATENT SPECIFICATION

(11) **EP 3 206 685 B1**
(45) Date of publication and mention of the grant of the patent: **08.05.2024**
(21) Application number: 15850725.1
(22) Date of filing: 15.10.2015
(51) Int. Cl.: A61K 31/4174, A61K 31/4184, A61K 35/38, A61P 29/00

(54) **PREVENTION OR TREATMENT OF SLEEP DISORDERS USING DEXMEDETOMIDINE FORMULATION**
PRÄVENTION ODER BEHANDLUNG VON SCHLAFSTÖRUNGEN MIT DEXMEDETOMIDINFORMULIERUNG
PRÉVENTION OU TRAITEMENT DE TROUBLES DU SOMMEIL AU MOYEN D'UNE FORMULATION DE DEXMÉDÉTOMIDINE

(30) Priority: 15.10.2014 US 201462064205 P
(43) Date of publication of application: 23.08.2017
(73) Proprietor: BioXcel Therapeutics, Inc., New Haven, CT 06511 (US)
(72) Inventor: NEGI, Harsh, New Delhi 110058 (IN); SAINI, Deepa, Uttar Pradesh 201105 (IN); SHARMA, Sameer, Himachal Pradesh 176061 (IN); NANDABALAN, Krishnan, Guilford, Connecticut 06437 (US); YOCCA, Frank, Killingworth, Connecticut 06415 (US)
(74) Representative: Cooley (UK) LLP
(86) International application number: PCT/US2015/055828
(87) International publication number: WO 2016/061413

(56) References cited:
- WO-A1-2005/032519
- WO-A1-2012/177326
- WO-A2-2013/090278
- WO-A2-2015/054059
- US-A1- 2005 222 270
- US-A1- 2006 069 086
- US-A1- 2006 069 086
- US-A1- 2009 076 156
- US-A1- 2010 196 286
- US-A1- 2010 196 286
- US-A1- 2011 021 588
- US-A1- 2011 021 588
- US-A1- 2013 116 215
- MARKKU ANTTILA ET AL: "Bioavailability of dexmedetomidine after extravascular doses in healthy subjects", BRITISH JOURNAL OF CLINICAL PHARMACOLOGY, BLACKWELL SCIENTIFIC PUBL, GB, vol. 56, no. 6, 1 December 2003 (2003-12-01), pages 691-693, XP008155312, ISSN: 0306-5251, DOI: 10.1046/J.1365-2125.2003.01944.X [retrieved on 2003-08-29]
- NEHA PARIKH ET AL: "Single-Dose Pharmacokinetics of Fentanyl Sublingual Spray and Oral Transmucosal Fentanyl Citrate in Healthy Volunteers: A Randomized Crossover Study", CLINICAL THERAPEUTICS, ELSEVIER, AMSTERDAM, NL, vol. 35, no. 3, 15 February 2013 (2013-02-15), pages 236-243, XP028523226, ISSN: 0149-2918, DOI: 10.1016/J.CLINTHERA.2013.02.017 [retrieved on 2013-02-27]

## Description

### FIELD OF INVENTION

The present invention relates to a sublingual formulation of dexmedetomidine. The present invention also relates to a method for treatment of a disease or condition which is susceptible to treatment with a α2-adrenoceptor agonist, e.g., a sleep disorder, in a patient in need thereof including administering an effective amount of a sublingual dexmedetomidine formulation or pharmaceutically acceptable salt thereof, solvate thereof, or derivative thereof.

### BACKGROUND/PRIOR ART OF INVENTION

Sleep disorders e.g., insomnia, affect millions of people. It is estimated that insomnia affects about 60-70 million Americans. Insomnia disorder can have a significant negative impact on quality of life, compromising the health, general well-being, and/or safety of the person suffering from insomnia.

Insomnia involves a persistent inability to fall asleep or persistent difficulty in falling asleep and/or remaining asleep during normal sleep times.

Medical literature has recognized four types of insomnia, including sleep onset insomnia (e.g., trouble falling asleep at bedtime), sleep maintenance insomnia (e.g., disturbed sleep during the night), early morning awakening, and transient insomnia (e.g., new environment, first night in hotel syndrome). Other types of insomnia include "middle-of-the-night" insomnia, "late night" insomnia, "prolonged awakening after sleep onset" insomnia, "sleep maintenance" insomnia, and insomnia that follows after "middle-of-the-night" awakening, each of which has a component of interrupted sleep.

Insomnia may be caused by, for example, certain drugs and/or stimulants (e.g., caffeine), hormonal fluctuations, stress, anxiety, depression, and/or neurological disorders, among other factors.

Generally, sedative hypnotic drugs such as benzodiazepines have been used to treat insomnia for many years. Examples of benzodiazepines include temazepam (e.g., Restoril^{®}), flunitrazepam (e.g., Rohypnol^{®}), triazolam (e.g., Halcion^{®}), flurazepam (e.g., Dalmane^{®}), nitrazepam (e.g., Mogadon^{®}), and midazolam (e.g., Versed^{®}). Prolonged administration of benzodiazepines and opioids causes tolerance and physical dependence. Non-benzodiazepine agents are also used to treat insomnia and include, for example, zolpidem, zaleplon and eszopiclone. In some cases, the antihistamine diphenhydramine (e.g., Benadryl^{®}) has been used as a sleep aid. Diphenhydramine is available over the counter and does not seem to induce dependence, but its effectiveness may decrease over time. Additionally, it may result in next-day sedation.

In view of the prevalence of insomnia disorders in the world population and serious draw backs of current therapy, it would be desirable to provide a new drug composition and method for treating insomnia. The patent application US-20100196286 discloses a method for fast, efficient treatment of insomnia, in a subject, the method comprising: administering a therapeutically effective amount (a dosage in the range of 0.02 µg/kg to 500 µg/kg) of an α2-adrenergic agonist (dexmedetomidine) composition to the subject using an injectable route.

The patent application WO-2012177326 discloses a biocompatible film comprising a single layer for a wide range of bioactive agents e.g. dexmedetomidine. Used as sublingual dosage form, e.g (solid) film, but not specific for use in treating sleep disorders.

The patent application WO-2010132882 discloses the administration of 50 ug or 100 ug of dexmedetomidine sublingually and IV in 24 healthy subjects. The sublingual formulations were administrated with 50 uL spray pump with a single or 2 pump actuations, providing a sublingual spray with drug delivery occurring via the oral transmucosal route. The hypotentive effects observed were so severe in some subjects (loss of consciousness, dizziness and postural hypotension), that a rescue medicine (IV saline treatment) needed to be administrated. In view of the side effects the inventors eliminated the 100 ug dose in Period 2 and subsequent Periods of the study. However, even with the lower 50 ug sub dose, hypotension was still a persistent side effect in the period 2 studies.

Currently, dexmedetomidine is only commercially available as an injectable formulation indicated for sedation, and it must be administered intravenously by a heath care professional. However, Dexmedetomidine formulation which could be used without hospital set ups and with self-administration for treatment of insomnia and other sleep disorders is not commercially available.

The present invention is directed to overcoming these and other deficiencies in the prior art.

### SUMMARY OF THE INVENTION

There is a demand for a pharmaceutical formulation for treatment of insomnia without drug abuse and tranquilizing effect.

The present invention discloses a sublingual film formulation for use in treating sleep disorder comprising about 0.1 mg to about 5 mg of dexmedetomidine or a pharmaceutically acceptable salt thereof formulated for delivery of dexmedetomidine across a subject's oral mucosa.

Thus, to solve the above-described problems, and with the expectation, the present invention provides a composition comprising a therapeutically effective amount of dexmedetomidine or a pharmaceutically acceptable salt thereof, solvate thereof, or derivative thereof formulated for delivery of dexmedetomidine across a subject's oral mucosa.

In one aspect, the composition is formulated as a sublingual formulation of dexmedetomidine wherein a dosage unit of the formulation is placed under the tongue, and the active component is absorbed through the surrounding mucous membranes. This results in a very fast onset of action.

In one of the aspect, the present invention provides a single-dose composition via sublingual administration, comprising a pharmacologically effective amount of dexmedetomidine or pharmaceutically acceptable salts thereof, solvates thereof, or derivatives thereof.

In another aspect, the invention relates to a pharmaceutical formulation for sublingual administration and process of preparing such formulation.

In yet another aspect, the compositions of the present invention are useful for the treatment of acute and chronic insomnia.

In another aspect of the present invention, the composition is used for targeting primary insomnia, wherein the predominant complaint is difficulty in initiating or maintaining sleep.

In another aspect of the present invention, the composition is used for patients suffering from frontotemporal dementia (FTD).

In another aspect of the present invention, the composition is used for treatment of insomnia associated with agrypnia excitata and/or hyperarousal.

A further aspect of the present invention relates to a method of treating or preventing a disease or condition which is susceptible to treatment with a α2-adrenoceptor agonist including selecting a patient with a disease or condition which is susceptible to treatment with a α2-adrenoceptor agonist, and administering to the patient a therapeutically effective amount of dexmedetomidine or a pharmaceutically acceptable salt thereof or derivative thereof.

A further aspect of the present invention is a method for treating or preventing a sleep disorder comprising selecting a patient with a sleep disorder and administering to the patient a therapeutically effective amount of dexmedetomidine. In one embodiment, administering is carried out sublingually.

Accordingly, the present technology discloses herewith a sublingual formulation of dexmedetomidine which can be used for treatment of insomnia by once in 24 hour intake and convenient for self-administration. The present formulation acts very fast without any drug abuse potential.

### BRIEF DESCRIPTION OF FIGURES

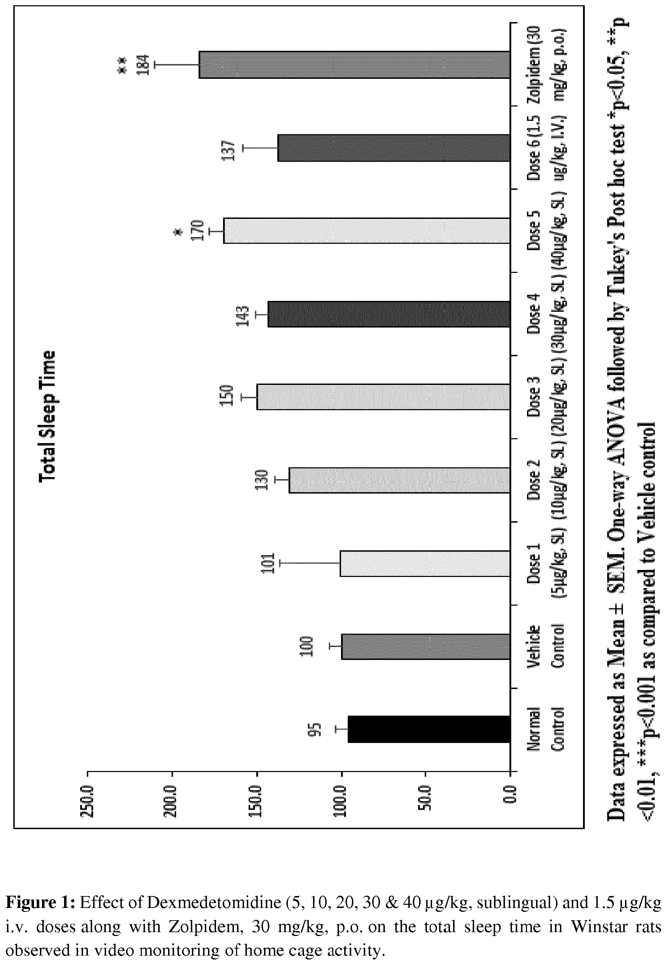

Figure 1. Effect of Dexmedetomidine (5, 10, 20, 30 & 40 µg/kg, sublingual) and 1.5 µg/kg i.v. doses along with Zolpidem, 30 mg/kg, p.o. on the total sleep time in Wistar rats observed in video monitoring of home cage activity.

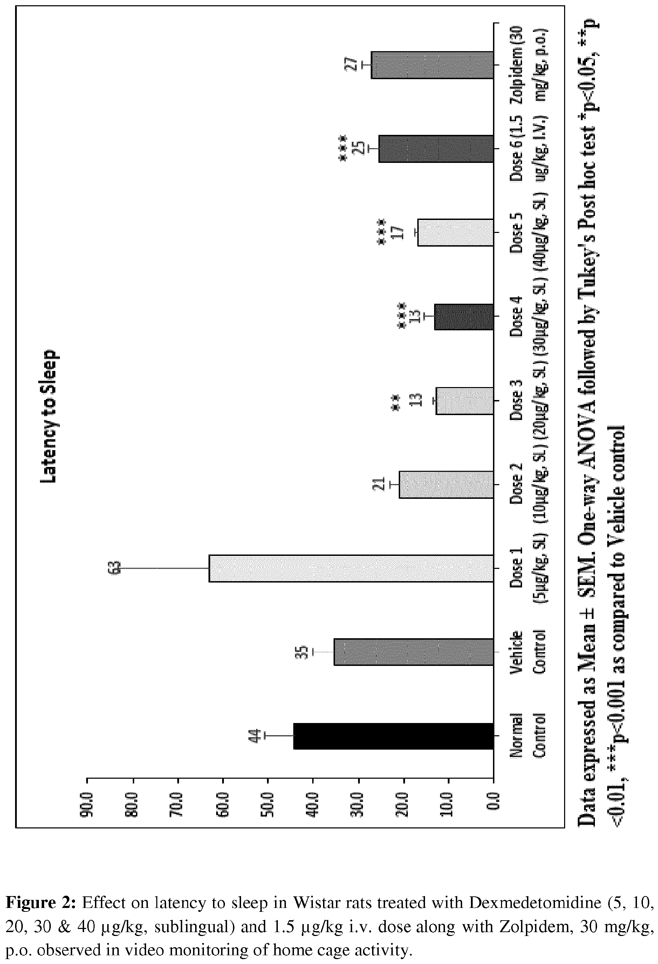

Figure 2. Effect on latency to sleep in Wistar rats treated with Dexmedetomidine (5, 10, 20, 30 & 40 µg/kg, sublingual) and 1.5 µg/kg i.v. dose along with Zolpidem, 30 mg/kg, p.o. observed in video monitoring of home cage activity.

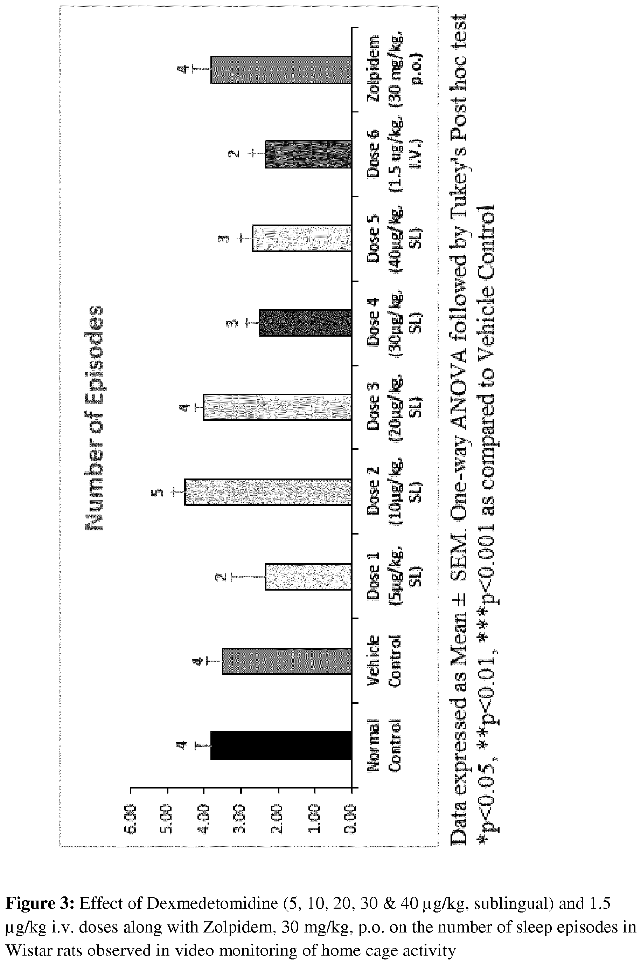

Figure 3. Effect of Dexmedetomidine (5, 10, 20, 30 & 40 µg/kg, sublingual) and 1.5 µg/kg i.v. doses along with Zolpidem, 30 mg/kg, p.o. on the number of sleep episodes in Wistar rats observed in video monitoring of home cage activity.

### DETAILED DESCRIPTION OF INVENTION

Dexmedetomidine is a specific α2-adrenergic receptor agonist that causes sedation and anaesthesia in mammals. In humans, dexmedetomidine is commercially available for sedation of initially intubated and mechanically ventilated patients during treatment in an intensive care setting, as well as sedation of non-intubated patients prior to or during surgical and other procedures. The present invention discloses that dexmedetomidine is very effective for treatment of sleep disorders, e.g., insomnia, and without causing drug dependence.

Dexmedetomidine is commercially available as an injectable formulation. Injectable are not always the preferred mode of treatment for insomnia.

Dexmedetomidine could be absorbed from the oral cavity. However, oral dexmedetomidine via gastric route is not preferable due to high first pass metabolism. Metabolism of the drug leads to low systemic bioavailability and greater variability in response.

In particular, in oral administration via the gastric route, the drug has to pass through the gastrointestinal system (portal circulation) in order to enter the blood stream. The time to achieve a therapeutic effect may be quite long, typically around 45 minutes or longer. In view of this, onset of action can be delayed in many patients, leading to a frustrating lack of "on demand" sleep, and possibly to undesirable residual effects the following day.

Hence, the present invention provides herein new compositions of dexmedetomidine or pharmaceutically acceptable salts, solvates, or derivatives thereof formulated for delivery of dexmedetomidine across a subject's oral mucosa and, in certain embodiments, methods of use thereof in the prevention, treatment, and management of sleep disorders, e.g. Insomnia. Delivery via the mucous membranes is very fast and avoids first pass metabolism. The minimum dose is effective to treat insomnia and quick onset of action. Moreover, the mucous membrane of the oral cavity is highly vascular and well supplied with lymphatic drainage sites.

Oral transmucosal administration of the drug also results in an enhanced rate of absorption as compared to an oral ingestion (portal circulation), and thereby results in a vastly increased bioavailability in a short period of time.

Pharmacologically dexmedetomidine is α2-adrenoceptor agonist. It can impact on the locus ceruleus centre of the brain synthesizing Norepinephrine and other awakening pathway activity to regulate the body wakefulness. Dexmedetomidine reduces the cerebral cortical hyper arousal level in patients with insomnia by changing the activity of Locus ceruleus-noradrenergic system and other arousal pathways of ascending reticular activating system. It helps to restore the sleeping-awakening system and improve the symptoms of insomnia. Dexmedetomidine induces a qualitatively similar pattern of c-fos expression as seen during normal NREM sleep, a decrease in the locus ceruleus and tuber mammillary nucleus and an increase in the ventrolateral preoptic nucleus (VLPO).

In another embodiment, the present invention discloses a pharmaceutical composition comprising a therapeutically effective amount of dexmedetomidine or a pharmaceutically acceptable salt, solvate, or derivative thereof in a non-injectable formulation and pharmaceutically acceptable carrier(s).

In one embodiment, the pharmaceutical composition is formulated for delivery of dexmedetomidine across a subject's oral mucosa, for example, as a tablet, capsule, patch, or oral spray.

In one embodiment, the pharmaceutical composition includes sublingual compositions of dexmedetomidine or a pharmaceutically acceptable salt, solvate, or derivative (e.g., pro-drug) thereof, such as in an amount sufficient to treat insomnia with a pharmaceutically acceptable excipient, as well as optional flavouring agents, preservatives, excipients, emulsifiers, buffers, colorants, and the like.

A further embodiment of the present invention relates to a method of treating or preventing a disease or condition which is susceptible to treatment with a α2-adrenoceptor agonist including selecting a patient with a disease or condition which is susceptible to treatment with a α2-adrenoceptor agonist, and administering to the patient a therapeutically effective amount of dexmedetomidine or a pharmaceutically acceptable salt thereof, solvate thereof, or derivative thereof.

Suitable diseases and conditions include, but are not limited to, sleep disorders, such as insomnia, snoring, sleep apnea, sleep deprivation, and restless legs syndrome, central nervous system disorders and/or states are not limited to headache, pain, epilepsy, convulsions, neurodegenerative diseases, Parkinson's disease, Alzheimer's disease, ataxias, dystonias, movement disorders, memory disorders, depression, avoidant personality disorder, anxiety, panic disorder, obsessive-compulsive disorders, phobias, impulsive disorders, cognitive disorders, mood disorders, psychoses, schizophrenia, drug abuse, chemical dependencies, drugs tolerance or withdrawal, posttraumatic stress syndrome and eating disorders.

In yet another embodiment, the present invention relates to a method of treating or preventing a sleep disorder including selecting a patient with a sleep disorder, and administering to the patient a therapeutically effective amount of dexmedetomidine or a pharmaceutically acceptable salt thereof, solvate thereof, or derivative thereof.

In one particular embodiment, the present invention also provides a method of treating insomnia comprising the steps of selecting a patient with insomnia and administering a sublingual pharmaceutical composition to the patient comprising a therapeutically effective dosage of dexmedetomidine or a pharmaceutically acceptable salt thereof, solvate thereof, or derivative a pharmaceutically acceptable excipient, wherein the dexmedetomidine or pharmaceutically acceptable salt thereof, solvate thereof, or derivative thereof is absorbed through said sublingual route.

In another embodiment, the dosage of dexmedetomidine or pharmaceutically acceptable salt thereof is based on the serum concentration which induces sleep i.e. approximately 0.5 to 1.2ng/ml. The total dose required is estimated to be in a range of 0.02 to 5 mg. This dose is for a human having a weight ranging from 40 to 100 kg. The human doses include 0.02 mg/day, 0.1 mg/day, 0.2 mg/day, 2mg/day, 3 mg/day, 4mg/day and 5 mg/day.

In another embodiment, a method is provided for treating insomnia, including administering to the oral mucosa of a mammal a systemically absorbed pharmaceutical composition comprising dexmedetomidine, or a pharmaceutically acceptable salt, solvate, or derivative thereof, in an amount effective to treat or to prevent insomnia in said mammal upon administration, wherein the pharmaceutical composition provides a physiologically active amount of dexmedetomidine into the systemic circulatory system of said mammal.

In yet another embodiment, a pharmaceutical composition is comprising dexmedetomidine, or a pharmaceutically acceptable salt, solvate, or derivative thereof, with a pharmaceutically acceptable excipient, wherein said pharmaceutical composition being configured and adapted for oral transmucosal administration to a mammal by applying said pharmaceutical composition to a mucous membrane of said mammal. Still further, the pharmaceutical composition may be configured and adapted for sublingual administration by applying said composition to a mucous membrane under the tongue of said mammal.

In yet another embodiment of the present invention, a method for administering dexmedetomidine or a pharmaceutical acceptable salt, solvate, or derivative thereof to a mammal includes spraying the oral mucosa of the mammal with spray composition comprising dexmedetomidine or a pharmaceutically acceptable salt, solvate, or derivative thereof in a pharmaceutically acceptable liquid vehicle.

In certain embodiments, the dexmedetomidine products described herein are pharmaceutical formulations for the treatment of sleep disorders, e.g., insomnia.

As used herein, the term "pharmaceutically acceptable" includes those compounds, materials, compositions, dosage forms, and methods of use thereof that are within the scope of sound medical judgment and suitable for use in contact with the tissues of human beings and animals without excessive toxicity, irritation, allergic response, or other problem or complication, while being commensurate with a reasonable benefit/risk ratio and eliciting a desired pharmacological response.

In certain other embodiments, the pharmaceutical formulation of present invention is also useful for patients suffering from chronic pain having additional difficulty due to insomnia and sleeping disorders. Back pain is the most common type of chronic pain problem, and is the most prevalent medical disorder in industrialized societies. Not surprisingly, individuals with chronic back pain problems frequently report significant interference with sleep. It has been reported that two-thirds of patients with chronic back pain suffered from sleep disorders. Research has demonstrated that disrupted sleep will, in turn, exacerbate the chronic back pain problem. Thus, a vicious cycle develops in which the back pain disrupts one's sleep, and difficulty in sleeping makes pain worse, which in turn makes sleeping more difficult.

In yet another embodiment, the pharmaceutical formulation of present invention is also useful for the patients having difficulty falling asleep, and awakening frequently during the night.

In some embodiments, the pain could be neuralgia, myalgia, hyperalgia, hyperpathia, neuritis, or neuropathy. In some embodiments, the pain is associated with or caused by cancer, viral infection, physical trauma, arthritis, headache, migraine, or lower back pain. In some embodiments, the physical trauma is associated with or caused by surgery, a burn, blunt force trauma, or other trauma that can cause pain, such as being in an accident.

In one another embodiment, the pharmaceutical formulation of present invention is also useful for patients suffering from insomnia. Several psychological and physiological factors contribute to the onset and perpetuation of insomnia, such as anxious-ruminative personality traits, stressful events, age-related sleep homeostasis weakening mechanisms, menopause and biologic - genetic diathesis of central nervous system hyperarousal.

In one another embodiment, the pharmaceutical formulation of present invention is also useful for patients suffering from frontotemporal dementia (FTD). Frontotemporal dementia or frontotemporal degenerations refers to a group of disorders caused by progressive nerve cell loss in the brain's frontal lobes (the areas behind your forehead) or its temporal lobes (the regions behind your ears). The nerve cell damage caused by frontotemporal dementia leads to loss of function in these brain regions, which variably cause deterioration in behaviour and personality, language disturbances, or alterations in muscle or motor functions.

In one another embodiment, the pharmaceutical formulation of present invention is also useful for patients suffering from insomnia with agrypnia excitata and/or hyperarousal disorder in the indications selected from the group comprising of multiple system atrophy, creutzfeldt-jakob disease, corticobasal degeneration, frontotemporal dementia, gerstmann-straussler-scheinker syndrome, huntington disease, fatal familial insomnia, cushing's syndrome, hypercortisolism, neurofibromatosis type 1, norrie disease, progressive supranuclear palsy, hereditary spastic paraplegia, alpers syndrome, Slos, fragile x syndrome, mulvihill-smith syndrome, transmissible spongiform encephalopathy, morvan syndrome, morvan's fibrillary chorea, peripheral nerve hyperexcitability, CAPS and PTSD

Dexmedetomidine contains a basic nitrogen atom capable of forming pharmaceutically acceptable salts with pharmaceutically acceptable acids. The term "pharmaceutically acceptable salts" in this respect refers to the relatively non-toxic, inorganic, and organic acid addition salts of dexmedetomidine. These salts may be prepared in situ during final isolation and purification of dexmedetomidine or by separately reacting purified dexmedetomidine in its free base form with a suitable organic or inorganic acid, and thereafter isolating the salt thus formed. Furthermore, the salt may be formed during a manufacturing process to produce formulation. The salts of dexmedetomidine comprises and are not limited to the hydrohalide (including hydrobromide and hydrochloride), sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, 2-hydroxyethylsulfonate, and laurylsulphonate salts, and the like.

Dexmedetomidine derivatives may include covalent modifications that create a pro-drug. Upon administration, the pro-drug derivative undergoes chemical modification by the mammal that yields dexmedetomidine.

The sublingual formulations of dexmedetomidine described herein are intended for administration directly to the mucosa (e.g., the oral mucosa in a mammal). Drug delivery occurs substantially via the oral transmucosal route and not via swallowing followed by gastrointestinal absorption. The term "transmucosal" refers to delivery across or through a mucosal membrane. In particular," oral transmucosal" delivery of a drug includes delivery across any tissue of the mouth, pharynx, larynx, trachea, or upper gastrointestinal tract, particularly the sublingual, buccal, gingival and palatal mucosal tissues.

The term "sublingual" literally means "under the tongue" and refers to a method of administering substances via the mouth in such a way that the substances are rapidly absorbed via the blood vessels under the tongue rather than via the digestive tract. Sublingual absorption occurs through the highly vascularized sublingual mucosa, which allows a substance direct access to the blood circulation, thereby providing for direct systemic administration independent of gastrointestinal influences and avoiding undesirable first-pass hepatic metabolism. As compared to other routes of administration, transmucosal absorption of dexmedetomidine in the present formulations may have a significantly faster onset with greater bioavailability, which is required to treat different types of insomnia as described above.

Pharmaceutical formulations of the present invention may be administered to mammals, including humans, as well as human companion animals (e.g., rat cats and dogs), agricultural livestock, and other animals in need thereof.

The sublingual formulations of dexmedetomidine described herein may be co administered with other medicines, including NSAIDS such as aspirin, ibuprofen, naproxen, celecoxib, acetaminophen, and other cyclooxygenase inhibitors; opioids such as codeine, oxycodone, morphine, methadone, and fentanyl; anticonvulsants and anti-arrhythmics such as phenytoin and carbamazepine; and antidepressants such as amitriptyline, imipramine, venlafaxine, clonidine and other active α-2 receptor agonist compounds. In particular, dexmedetomidine may significantly potentiate the effectiveness of opioids, permitting a reduction in required opioid dosage while maintaining equivalent therapeutic usefulness.

By "sleep on demand", we include that the formulation consistently induces sleep, i.e. in at least 90% of cases (on an intra- and/or inter-patient basis), within 60 minutes, preferably within 45 minutes, more preferably within 3 minutes.

In another embodiment of present invention, the drugs levels are maintained at >about 0.3 microgram/ml for 2 to 6 hrs.

The disclosed embodiments are not intended as limiting. In practicing the invention, formulations containing dexmedetomidine may alternatively or additionally be provided as other sublingual and/or buccal compatible dosage forms.

### Definitions

The terms "composition of the invention", "pharmaceutical formulation", and equivalent expressions, are meant to embrace compounds as hereinbefore described, which expression includes the prodrugs, the pharmaceutically acceptable salts, the oxides, the solvates, e.g. hydrates, and inclusion complexes of that compound, where the context so permits, as well as a mixture of any such forms of that compound in any ratio. Inclusion complexes are described in Remington, The Science and Practice of Pharmacy, 19th Ed. 1:176-177 (1995). The most commonly employed inclusion complexes are those with cyclodextrins, and all cyclodextrin complexes, natural and synthetic, are specifically encompassed within the claims. Thus, in accordance with some embodiments of the invention, a compound as described herein, including in the contexts of pharmaceutical compositions, methods of treatment, and compounds per se, is provided as the salt form. Similarly, reference to intermediates, whether or not they themselves are claimed, is meant to embrace their salts, and solvates, where the context so permits. For the sake of clarity, particular instances when the context so permits are sometimes indicated in the text, but these instances are purely illustrative and it is not intended to exclude other instances when the context so permits.

Pharmaceutical Formulations: Pharmaceutical formulations for the administration of dexmedetomidine formulation to treat insomnia with the method of the present invention may take the form of transdermal patches, transbuccal patches, nasal inhalant forms, mucosal spray, orally administered tablets and capsules, and tablets or lozenges, or "lollipop" formulations for administration through the oral mucosal tissue. The latter formulations include tablets, lozenges and the like which are dissolved while being held on or under the tongue, or in the buccal pouch.

A "pharmaceutically acceptable" carrier or excipient, as used herein, means approved by a regulatory agency of the Federal or a state government, or as listed in the U.S. Pharmacopoeia or other generally recognized pharmacopoeia for use in mammals, and more particularly in humans.

The term "pharmaceutical composition" as used in accordance with the present invention relates to compositions that can be formulated in any conventional manner using one or more pharmaceutically acceptable carriers or excipients.

The term "solvate" refers to a composition of the present technology in the solid state, wherein molecules of a suitable solvent are incorporated in the crystal lattice. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions.

The term "derivative" refers, for example, to crystal forms, polymorphs, or prodrugs of the compositions useful according to the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compositions of the invention. The term "prodrug" means compounds that are rapidly transformed in vivo to yield the parent compound, for example by hydrolysis in blood. Commonly, the conversion of prodrug to drug occurs by enzymatic processes in the liver or blood of the mammal. Compositions of the invention may be chemically modified without absorption into the systemic circulation, and in those cases, activation in vivo may come about by chemical action (as in the acid-catalysed cleavage in the stomach) or through the intermediacy of enzymes and microflora in the gastrointestinal Gl tract. The composition may bear metabolically cleavable groups, which have the advantage that they may exhibit improved bioavailability as a result of enhanced solubility and/or rate of absorption conferred upon the parent compound by virtue of the presence of the metabolically cleavable group. A thorough discussion of prodrugs is provided in the following: Design of Prodrugs, H. Bundgaard, ed., Elsevier (1985); Methods in Enzymology, K. Widder et al, Ed., Academic Press, 42, p.309-396 (1985); A Textbook of Drug Design and Development, Krogsgaard-Larsen and H. Bundgaard, ed., Chapter 5; "Design and Applications of Prodrugs," p.113-191 (1991); Advanced Drug Delivery Reviews, H. Bundgaard, 8, p.1-38 (1992); Journal of Pharmaceutical Sciences, 77:285 (1988); Nakeya et al, Chem. Pharm. Bull., 32:692 (1984); Higuchi et al., "Pro-drugs as Novel Delivery Systems," Vol. 14 of the A.C.S. Symposium Series, and Bioreversible Carriers in Drug Design, Edward B. Roche, ed., American Pharmaceutical Association and Pergamon Press (1987). Examples of prodrugs include, but are not limited to, acetate, formate, and benzoate derivatives of amine functional groups in the compositions of the invention.

As used herein, the term "human" or "patient," used interchangeably, means any animal, including mammals, such as mice, rats, other rodents, rabbits, dogs, cats, swine, cattle, sheep, horses, or primates, such as humans.

As used herein, the term "sedation" means depressed consciousness in which a patient or subject retains the ability to independently and continuously maintain an open airway and a regular breathing pattern, and to respond appropriately and rationally to physical stimulation and verbal commands.

The term "dosage" is intended to encompass a formulation expressed in terms of µg/kg/hr, µg/kg/day, mg/kg/day, or mg/kg/hr.

A "dose" is an amount of an agent administered to a patient in a unit volume or mass, e.g., an absolute unit dose expressed in mg of the agent. The dose depends on the concentration of the agent in the formulation, e.g., in moles per litre (M), mass per volume (m/v), or mass per mass (m/m).

The term "drug addiction" means dependence on an illegal drug, controlled substance or a medication. When you're addicted, you may not be able to control your drug use and you may continue using the drug despite the harm it causes. Drug addiction can cause an intense craving for the drug. You may want to quit, but most people find they can't do it on their own.

The term "method of treating" means amelioration or relief from the symptoms and/or effects associated with the disorders described herein. As used herein, reference to "treatment" of a patient is intended to include prophylaxis.

In another embodiment, the present invention unexpectedly discloses that a sublingual formulation of dexmedetomidine, or pharmaceutically acceptable salts thereof, and/or derivatives thereof can be developed for the quick relief of insomnia corresponding to Cplasma, a Cmax, and a reduced time to Tmax,

Methods of formulating the composition for sublingual administration in a tablet form are well known to those skilled in the art. Any such method may be used to formulate the composition of the present invention. For example, the dexmedetomidine may be formulated as a blend of drug powder with other ingredients, as granules or tablet or microspheres.

The word "sedative" means a drug that calms a patient, easing agitation and permitting sleep. Sedatives generally work by modulating signals within the central nervous system.

The following examples are intended to be illustrative and are not according to the claimed invention.

Example 1: Composition for a typical tablet formulation used for sublingual or buccal delivery. Buccal delivery may require a back-liner if specified.

| | Per unit (% w/w) | | | |
|---|---|---|---|---|
| Dexmedetomidine | 0.05% to 5% | 0.05% to 5% | 0.05% to 5% | 0.05% to 5% |
| Povidone (and)/(or) Hypromellose | 1.0 - 10.0 % | 1.0 - 10.0 % | - | - |
| Crospovidone (and) / (or) Croscarmellose Sodium (and) / (or) Sodium starch glycollate | 5 - 10% | 5 - 10% | 5 - 10% | 5 - 10% |
| Sucralose (and) / (or) Aspartame | 0.1 - 4.0 % | 0.1 - 4.0 % | 0.1 - 4.0 % | 0.1 - 4.0 % |
| Magnesium Stearate (and)/ (or) Silicon dioxide | 0.1 - 1.0 % | - | 0.1 - 1.0 % | - |
| Lactose Monohydrate (and) / (or) Mannitol or cellulose | q.s. 100% | q.s. 100 % | q.s. 100 % | q.s. 100 % |

Manufacturing process for a sublingual Dexmedetomidine tablet:

Any traditional tablet manufacturing method like direct compression, wet granulation or dry granulation can be used.

In a direct compression method, all materials including drug substance should be sifted in a specific sequence as per process optimized and then blended in V-blender or any other suitable blender sequentially to achieve uniform mixing of drug substance in the blend. The blend is then further compressed into tablets using appropriate tooling.

In a wet granulation method, the drug substance alone or with other excipients such binder, sweetener and others shall be dissolved/ dispersed into suitable solvent (water or other acceptable one). The same shall be used to granulate the sifted blend of other ingredients. The granules are then dried and sized appropriately. The granules are then lubricated in a suitable blender and compressed into tablets of specific dimensions using appropriate tooling.

In a dry granulation method, the granules will be prepared either by compaction (eg. Roller compactor) or slugging or any other suitable technique and tablets will be prepared in a similar manner to that of wet granulation technique.

Other sublingual dosage forms such as films, buccal patches or spray formulations, will involve appropriate fabrication technology like usage of film casting equipment for preparing a film dosage form or usage of an impermeable or semi-permeable back liner for buccal patch dosage form or filling into canisters for spray dosage forms.

### Example 2:

### Evaluation of sleep promoting properties of sublingual dexmedetomidine in wistar rats

Sublingual dexmedetomidine formulation of present invention was tested in rats for its ability to increase the amount of sleep or decrease sleep interruption or both without undesired effects. Test animals' activity was continuously monitored by using a video camera overhead and data analysed for latency of sleep, total sleep time and number of sleep episodes by using the Noldus Ethovision-XT 11.

Male Wistar rats were approximately of the weight 180g - 220g and age of about 5-7 weeks old. Animals were numbered and kept in acclimatization for a period of 5-7 days before the initiation of the experiment. All the experiments on animals were conducted in accordance with the guidelines of the Committee for the Purpose of Control and Supervision of Experiments on Animals (CPCSEA), Government of India the Association for Assessment and Accreditation of Laboratory Animal Care international (AAALAC).

Recording Environment: Animals were housed group wise (three animals per cage) within a microisolator cage modified with an inserted polycarbonate filter-top riser to allow more vertical headroom. Animals were maintained on the normal rodent chow ad libitum and given free access to fresh autoclaved potable drinking water. Animals were kept in a controlled environmental condition with 22±3°C temperature, 50±20% humidity, a light dark cycle of 12 hours each and 15-20 fresh air changes per hour.

Animal Groups: Wistar rats were randomly divided into different groups, each containing 6 animals. The study groups were divided as normal control group, vehicle control group, test compound groups and reference compound group.

Test Drug and Reference Drug: Test drug dexmedetomidine procured from Neon labs, India and reference drug zolpidem procured from Sigma Aldrich, Cat. No Z103 Sigma.

### Study Design and Dosing:

Animals were weight once at randomization and each day thereafter. The animals were studied from day 1 till day 9. On day 1, test and reference compound were administered and activity were recorded for 180 minutes using overhead video camera and the data was analysed with Noldus Ethovision-XT 11. On day 3, animals were dosed with test compounds and respiratory parameters such as tidal volume, frequency of respiration; heart rate and blood pressure were recorded. On day 5 and day 6, animals were trained on the rotarod apparatus (4-40 rpm for 300s). On day 7, animals were administered with test compounds and motor coordination was tested on rotarod at 3, 10, 17, 24 and 30 minutes post administration to establish a temporal scale of loss of motor activity and drowsiness. On day 9, animals were dosed with test compounds and blood was collected at 15, 30, 60 and 120 min post sublingual dosing to estimate plasma drug concentration of dexmedetomidine. All the animals were euthanized by CO₂ inhalation on day 9. The animal in the satellite groups was dosed with test compounds and blood was collected on day 0 at 15, 30, 60, and 120 min post sublingual dosing to estimate plasma drug concentration of dexmedetomidine after first drug exposure.

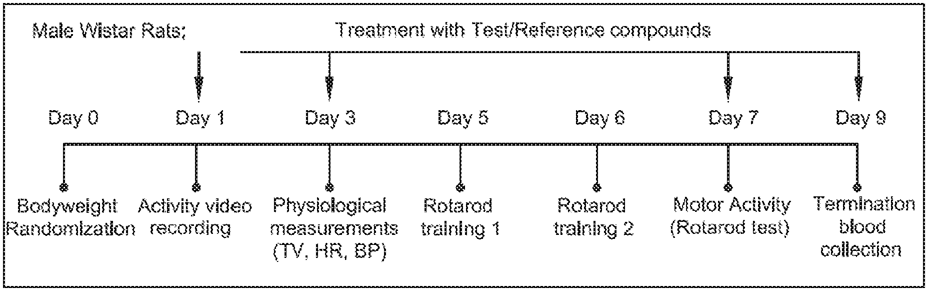

The normal control group received no treatment; vehicle control group received 0.9% saline water; the test group received compound (dexmedetomidine), administered sublingually in different doses (dose 1 - 5.14 µg/kg of body weight, dose 2 - 10 µg/kg of body weight, dose 3 - 20 µg/kg of body weight, dose 4 - 30 µg/kg of body weight and dose 5 - 40 µg/kg of body weight) and also administered parenterally (dose 6 - 1.5 µg/kg of body weight); and the reference compound (zolpidem) was administered orally in dose of 30 mg/kg of body weight. The treatment was given such that no rat received the same treatment twice.

Total sleep time, latency to sleep and number of sleep episodes were measured: After administration of each dose to each group of animal, the latency to sleep, total sleep time and sleep episodes was recorded.

### Results:

A comparison of total sleep time, latency to sleep and number of sleep episodes for different groups: normal control, vehicle control, dexmedetomidine and zolpidem at different doses was performed and is presented as tabulated in table 1 and table 2.

One way ANOVA test was applied for the comparison of the data from different groups for total sleep time, latency to sleep and number of sleep episodes.

**Table 1:**

| **Group** | | **Total Sleep time (min)** | **Total Sleep time Normalized to Vehicle*** | **Number of Sleep Episodes** | **Latency to Sleep** |
|---|---|---|---|---|---|
| 1 | Normal Control | 53 | 67 | 4 | 20 |
| 2 | | 71 | 89 | 3 | 40 |
| 3 | | 92 | 115 | 5 | 41 |
| 4 | | 94 | 118 | 3 | 55 |
| 5 | | 84 | 105 | 3 | 69 |
| 6 | | 62 | 78 | 5 | 40 |

| **Mean, SEM** | | **76.00** | **95.33** | **3.83** | **44.17** |
|---|---|---|---|---|---|
| 7 | Vehicle Control (0.9% Saline) | 57 | 72 | 5 | 22 |
| 8 | | 76 | 95 | 2 | 31 |
| 9 | | 84 | 105 | 3 | 52 |
| 10 | | 96 | 121 | 4 | 23 |
| 11 | | 93 | 117 | 4 | 43 |
| 12 | | 72 | 90 | 3 | 41 |

| **Mean, SEM** | | **79.67** | **100.00** | **3.50** | **35.33** |
|---|---|---|---|---|---|
| 13 | Dose 1 (5.14µg/kg Dexmedetomidine SL) | 87 | 184 | 2 | 97 |
| 14 | | 96 | 203 | 5 | 101 |
| 15 | | 59 | 125 | 2 | 87 |
| 16 | | 44 | 93 | 5 | 94 |
| 17 | | 0 | 0 | 0 | 0 |
| 18 | | 0 | 0 | 0 | 0 |

| **Mean, SEM** | | **47.67** | **100.83** | **2.33** | **63.17** |
|---|---|---|---|---|---|
| 19 | Dose 2 (10µg/kg Dexmedetomidine SL) | 102 | 128 | 5 | 18 |
| 20 | | 81 | 102 | 5 | 29 |
| 21 | | 86 | 108 | 5 | 21 |
| 22 | | 112 | 141 | 4 | 24 |
| 23 | | 121 | 152 | 5 | 16 |
| 24 | | 120 | 151 | 3 | 18 |

| **Mean, SEM** | | **103.67** | **130.33** | **4.50** | **21.00** |
|---|---|---|---|---|---|
| 25 | Dose 3 (20µg/kg Dexmedetomidine | 106 | 133 | 5 | 15 |
| 26 | | 139 | 174 | 3 | 12 |
| 27 | SL) | 114 | 143 | 4 | 13 |
| 28 | | 113 | 142 | 4 | 15 |
| 29 | | 144 | 181 | 4 | 10 |
| 30 | | 102 | 128 | 4 | 11 |

| **Mean, SEM** | | **119.67** | **150.17** | **4.00** | **12.67** |
|---|---|---|---|---|---|
| 31 | Dose 4 (30µg/kg Dexmedetomidine SL) | 129 | 162 | 4 | 11 |
| 32 | | 101 | 127 | 2 | 23 |
| 33 | | 126 | 158 | 2 | 8 |
| 34 | | 108 | 136 | 2 | 10 |
| 35 | | 126 | 158 | 3 | 16 |
| 36 | | 94 | 118 | 2 | 11 |

| **Mean, SEM** | | **114.00** | **143.17** | **2.50** | **13.17** |
|---|---|---|---|---|---|
| 37 | Dose 5 (40µg/kg Dexmedetomidine SL) | 144 | 181 | 2 | 16 |
| 38 | | 107 | 134 | 2 | 17 |
| 39 | | 148 | 186 | 4 | 16 |
| 40 | | 151 | 190 | 3 | 18 |
| 41 | | 127 | 159 | 2 | 13 |
| 42 | | 134 | 168 | 3 | 20 |

| **Mean, SEM** | | **135.17** | **169.67** | **2.67** | **16.67** |
|---|---|---|---|---|---|
| 43 | Dose 6 (1.5µg/kg Dexmedetomidine I.V.) | 85 | 180 | 3 | 18 |
| 44 | | 87 | 184 | 3 | 27 |
| 45 | | 45 | 95 | 3 | 26 |
| 46 | | 61 | 129 | 1 | 24 |
| 47 | | 84 | 177 | 2 | 85* |
| 48 | | 27 | 57 | 2 | 32 |

| **Mean, SEM** | | **64.83** | **137.00** | **2.33** | **25.40** |
|---|---|---|---|---|---|
| 43 | Zolpidem (30mg/kg) | 117 | 247 | 3 | 24 |
| 44 | | 123 | 260 | 3 | 24 |
| 45 | | 79 | 167 | 6 | 28 |
| 46 | | 97 | 205 | 4 | 26 |
| 47 | | 54 | 114 | 3 | 23 |
| 48 | | 52 | 110 | 4 | 37 |
| **Mean, SEM** | | **87.00** | **183.83** | **3.83** | **27.00** |

**Table 2:**

| **Group** | **Total Sleep Time (Mean and SEM)** | | **Latency to Sleep (Mean and SEM)** | | **Number of Episodes (Mean and SEM)** | |
|---|---|---|---|---|---|---|
| Normal Control | 95.33 | 8.44 | 44.2 | 6.74 | 3.83 | 0.4014 |
| Vehicle Control | 100 | 7.448 | 35.3 | 4.89 | 3.50 | 0.4282 |
| Dose 1 (5.14 µg/kg, SL) | 100.8 | 35.747 | 63.2 | 20.062 | 2.33 | 0.9189 |
| Dose 2(10µg/kg, SL) | 130.31 | 8.789 | 21.0 | 1.966 | 4.50 | 0.3416 |
| Dose 3 (20µg/kg, SL) | 150.2 | 8.987 | 12.7 | 0.8433 | 4.00 | 0.2582 |
| Dose 4(30µg/kg, SL) | 143.2 | 7.618 | 13.2 | 2.242 | 2.50 | 0.342 |
| Dose 5(40µg/kg, SL) | 169.67 | 8.558 | 16.7 | 0.9545 | 2.67 | 0.3333 |
| Dose 6 (1.5 µg/kg, I.V.) | 137 | 21.51 | 25.4 | 2.272 | 2.33 | 0.3333 |
| Zolpidem(30 mg/kg, p.o.) | 183.83 | 26.36 | 27.0 | 2.129 | 3.83 | 0.4773 |

### Conclusion:

**Total Sleep Time (total duration of sleep in 180 min.):** A significant increase in Total Sleep time for Sublingual dexmedetomidine treated groups was observed with significant effect at 40µg/kg. The reference compound Zolpidem, tested at the standard dose, also showed significant increase in sleep time as compared to NC and Vehicle control.

**Latency to Sleep (Time of sleep onset):** Latency to sleep was significantly lowered at 10, 20, 30 & 40 µg/kg dose of dexmedetomidine dosed sublingually and with parenteral dexmedetomidine (1.5 µg/kg, p.o.) as compared to vehicle control. Significant decrease in latency to sleep was observed at 10, 20, 30 and 40 µg/kg dose of dexmedetomidine, even though no significant increase in sleep time was observed at these doses. The reference compound Zolpidem also showed reduction in the sleep latency when compared to normal and vehicle control.

**Number of sleep episodes**: Number of sleep episodes was gradually decreased from 10, 20, 30 & 40 µg/kg doses of dexmedetomidine dosed sublingually and with parenteral dexmedetomidine (1.5 µg/kg p.o.) as compared to vehicle control. The reference compound zolpidem showed similar number of sleep episodes as the vehicle control.

Thus it can be concluded that sublingually delivered dexmedetomidine at a dose range of 10 to 40 micrograms/kg significantly reduced latency of sleep while not significantly affecting total sleep time. The number of sleep episodes gradually decreased in response to increased dosing of sublingual dexmedetomidine. The dose of 40 microgram/kg of sublingual dexmedetomidine also showed significant increase in total sleep time. Thus dexmedetomidine delivered sublingually significantly reduces latency to sleep.

## Claims

1. A sublingual film formulation for use in treating sleep disorder comprising about 0.1 mg to about 5 mg of dexmedetomidine or a pharmaceutically acceptable salt thereof formulated for delivery of dexmedetomidine across a subject's oral mucosa.

2. The formulation for use as in claim 1, wherein the sleep disorder is insomnia.

3. The formulation for use as in claim 1 or 2, wherein an effective time for treatment is in a range of from about 3 to about 30 minutes.

## Patentansprüche

1. Sublingualfilmformulierung zur Verwendung bei der Behandlung einer Schlafstörung, umfassend etwa 0,1 mg bis etwa 5 mg Dexmedetomidin oder ein pharmazeutisch unbedenkliches Salz davon, formuliert zur Zuführung von Dexmedetomidin über die Mundschleimhaut eines Individuums.

2. Formulierung zur Verwendung nach Anspruch 1, wobei es sich bei der Schlafstörung um Schlaflosigkeit handelt.

3. Formulierung zur Verwendung nach Anspruch 1 oder 2, wobei eine effektive Zeit für die Behandlung in einem Bereich von etwa 3 bis etwa 30 Minuten liegt.

## Revendications

1. Formulation de film sublingual pour une utilisation dans le traitement d'un trouble du sommeil comprenant environ 0,1 mg à environ 5 mg de dexmédétomidine ou d'un sel pharmaceutiquement acceptable correspondant formulé(e) pour une administration de dexmédétomidine à travers une muqueuse orale d'un sujet.

2. Formulation pour une utilisation selon la revendication 1, dans laquelle le trouble du sommeil est l'insomnie.

3. Formulation pour une utilisation selon la revendication 1 ou 2, dans laquelle une durée effective pour un traitement est d'environ 3 à environ 30 minutes.
